Europäisches Patentamt

European Patent Office

Office européen des brevets

(19)

(11) Numéro de publication : **0 688 790 A1**

# (12) DEMANDE DE BREVET EUROPEEN

(21) Numéro de dépôt : 95401316.5

(22) Date de dépôt : 07.06.95

(51) Int. Cl.⁶ : **C07K 14/155,** G01N 33/569, G01N 33/68

(30) Priorité : **09.06.94 FR 9407062**

(43) Date de publication de la demande :
**27.12.95 Bulletin 95/52**

(84) Etats contractants désignés :
**AT BE CH DE DK ES FR GB GR IE IT LI LU MC NL PT SE**

(71) Demandeur : **CENTRE NATIONAL DE LA RECHERCHE SCIENTIFIQUE (CNRS)**
**3 rue Michel Ange**
**F-75794 Paris Cédex 16 (FR)**

(72) Inventeur : **Avrameas, Alexandre**
**7 allée Croix du Mont**
**F-94400 Vitry-sur-Seine (FR)**
Inventeur : **Pancino, Gianfranco**
**14 Quai de la Marne**
**F-75019 Paris (FR)**
Inventeur : **Sibille, Pierre**
**20 bis avenue du Clos**
**F-94210 la Varenne (FR)**
Inventeur : **Sonigo, Pierre**
**23 rue Gutemberg**
**F-75015 Paris (FR)**
Inventeur : **Strosberg, Arthur Donny**
**66 rue de Javel**
**F-75015 Paris (FR)**

(74) Mandataire : **Orès, Bernard et al**
**Cabinet ORES**
**6, Avenue de Messine**
**F-75008 Paris (FR)**

(54) **Fragment peptidique spécifique du virus de l'immunodéficience féline (VIF), et son utilisation comme réactif de diagnostic**

(57) Ledit fragment peptidique issu de la protéine Env du virus de l'immunodéficience féline (VIF) de souche Wo (peptide P253), correspond aux positions 693-709 de ladite protéine Env et présente la séquence suivante : Leu-Gly-X-Asn-Gln-Asn-Gln-Phe-Phe-X-Lys-Val-Pro-Ser-Ala-, dans laquelle X représente une cystéine ou une sérine, conformément aux séquences ID N° 1 à 4.

EP 0 688 790 A1

Jouve, 18, rue Saint-Denis, 75001 PARIS

La présente invention est relative à un fragment peptidique spécifique du virus de l'immunodéficience féline (VIF), ainsi qu'à son utilisation comme réactif de diagnostic.

L'immunodéficience féline est due à un lentivirus, le virus de l'immunodéficience féline (VIF), qui présente une structure génétique similaire à celle des lentivirus des primates (VIH et VIS).

L'immunodéficience féline pose un problème important de santé vétérinaire, dans la mesure où un nombre important de chats infectés par le VIF a été décelé aux Etats-Unis, au Japon et en Europe (5 à 30 % des animaux).

Plusieurs isolats viraux indépendants ont été mis en évidence à travers le monde et un certain nombre de travaux pour mettre en évidence la structure des souches isolées ont été réalisés, notamment en ce qui concerne la souche américaine *Petaluma* [R.L. TALBOTT et al. (Natl. Acad. Sci. USA, 1989, 86, 5743-5747) ; T.R. PHILIPPS et al. (J. Virol., 1990, 64, 10, 4605-4613)], les souches japonaises (souches TM1 et TM2) [T. MIYAZAWA et al. (Arch. Virol., 1989, 108, 59-68)] ou les isolats suisses (FIVZ1 et FIVZ2) [S. MORIKAWA et al., (Virus Research, 1991, 21, 53-63)].

Les séquences nucléotidiques de trois clones proviraux, dérivés des isolats de VIF américains (souche *Petaluma*) ont été décrites (clones FIV34TF10, FIV14 et isolat PPR) [R.A. OLMSTED et al., (Proc. Natl. Acad. Sci. USA, 1989, 86, 2448-2452) ; T.R. PHILIPPS et al., 1990 ; R.L. TALBOTT et al., (Proc. Natl. Acad. Sci. USA, 1989, 86, 5743-5747) et comparées aux deux isolats suisses (S. MORIKAWA et al.). Cette comparaison a conduit S. MORIKAWA et al. à préciser la présence de certaines régions conservées et de certaines régions variables dans le gène env du VIF.

Des souches françaises ont également été isolées (souches Wo et Me) [MORAILLON A. et al., 1992, Vet. Mic., 31, 41-45, *In vitro properties and experimental pathogenic effect of three feline immunodeficiency viruses isolated from cats with terminal diseases*].

Ces différents résultats ont permis de mettre au point un certain nombre de tests, pour la détection des animaux séropositifs ; on peut citer notamment :

- les tests ELISA utilisant les protéines recombinantes Gag p24 ou p17 (Reid G. et al., 1991, AIDS, **5**, 1477-1483) ; de tels tests présentent l'inconvénient d'entraîner des réactions faussement négatives, les anticorps anti-Gag n'étant pas présents de manière constante (FURUYA et al., 1992, Arch. Virol., **124**, 355-361),
- les tests ELISA utilisant un lysat de virus entier, lié à un support solide et obtenu à partir de fibroblastes infectés par du VIF (O'Connor et al. J. Clin. Microbiol, 1989, , 474-479 ; Steinman et al., J. Gen. Virol., 1990, **71** ,701-706) ; de tels tests présentent l'inconvénient d'entraîner à la fois des réactions faussement négatives et des réactions faussement positives,
- les tests ELISA utilisant un peptide synthétique conjugué à la peroxydase (Synbiotics) de tels tests présentent également l'inconvénient d'entraîner à la fois des réactions faussement négatives et des réactions faussement positives,
- les tests ELISA utilisant des peptides synthétiques issus de la protéine Env, tels que décrits dans la Demande EP 0 577 458, correspondant à des épitopes ± conservés de ladite protéine Env, tels que :
  . un fragment incluant un segment de 37 aminoacides, dénommé SUI, qui correspond aux positions 253-289 de la séquence Env (souche Wo) ;
  . un fragment incluant un segment de 37 aminoacides, dénommé SU2, qui correspond aux positions 388-424 de la séquence Env, lequel segment contient au moins un épitope incluant la séquence : Trp$^{398}$-Glu-Trp-Arg-Pro-Asp-Phe-Glu-Ser-Glu-Lys$^{408}$ (peptide dénommé P240) ;
  . un fragment incluant un segment de 26 aminoacides, dénommé SU3, qui correspond aux positions 467-492 de la séquence de protéine Env du VIF Wo ;
  . un fragment ncluant un segment de 21 aminoacides, dénommé SU4, qui correspond aux positions 508-528 de la séquence de protéine Env du VIF Wo ;
  . un fragment incluant un segment de 35 aminoacides, dénommé SUS, qui correspond aux positions 572-606 de la séquence de protéine Env du VIF Wo ;
  . un fragment incluant un segment de 51 aminoacides, dénommé TM1, qui correspond aux positions 595-647 de la séquence de protéine Env du VIF Wo ;
  . un fragment incluant un segment de 31 aminoacides, dénommé TM2, qui correspond aux positions 681-711 de la séquence de protéine Env du VIF Wo, lequel segment contient un épitope incluant la séquence Gln$^{699}$-Asn-Gln-Phe-Phe-Cys-Lys$^{705}$ ou la séquence Cys$^{697}$-Asn-Gln-Asn-Gln-Phe-Phe-Cys-Lys$^{705}$ (peptide dénommé P237) ;
  . un fragment incluant un segment de 45 aminoacides, dénommé TM3, qui correspond aux positions 744-788 de la séquence de protéine Env du VIF Wo, lequel segment contient un épitope incluant la séquence : Gln$^{764}$-Leu-Gln-Glu-Trp-Glu-Asp-Trp-Val-Gly-Trp-Ile-Gly-Asn-Ile$^{778}$ ou la séquence Gln$^{763}$-Gln-Leu-

Gln-Glu-Trp-Glu-Asp$^{770}$ (peptide dénommé P241) ou la séquence Val$^{772}$-Gly-Trp-Ile-Gly-Asn-Ile-Pro$^{779}$ (peptide dénommé P242) ;

. un fragment Incluant un segment de 29 aminoacides, dénommé TM4, qui correspond aux positions 826-854 de la séquence de protéine Env du VIF Wo.

Parmi ces peptides SU et TM décrits dans la Demande EP 0 577 458, certains sont considérés comme des fragments universels (notamment ceux inclus dans SU2, TM2 et TM3), c'est-à-dire reconnaissant les anticorps produits par un VIF, s'il est présent, quelle que soit la souche et d'autres sont considérés comme des fragments spécifiques de souche Wo (notamment SU3 et TM4).

Bien que ce dernier test présente une sensibilité nettement améliorée par rapport aux autres tests précités, la Demanderesse a trouvé, qu'il était possible d'améliorer encore la sensibilité et la spécificité des tests ELISA, réalisés à partir de peptides synthétiques, de manière à effectivement atteindre une fiabilité de l'ordre de 100 %, afin de disposer d'un test particulièrement adapté aux tests vétérinaires de routine.

La présente invention a pour objet un fragment peptidique issu de la protéine Env du VIF Wo, qui correspond aux positions 693-709 de ladite protéine Env.

Ledit peptide présente la séquence suivante :
Leu-Gly-X-Asn-Gln-Asn-Gln-Phe-Phe-X-Lys-Val-Pro-Ser-Ala-, dans laquelle X représente une cystéine ou une sérine, conformément aux séquences ID N° 1 à 4, ci-après.

De manière inattendue, en sélectionnant un peptide plus long que le peptide P237, précédemment décrit, pour le diagnostic des infections à VIF, aucun résultat faussement positif ni aucun résultat faussement négatif n'a été détecté.

La différence de réactivité entre le peptide P237 (9 résidus) et le peptide P253 (15 résidus) peut être une conséquence d'un repliement différent de ces deux peptides ; en particulier, le peptide selon la présente invention (P253) peut mieux imiter la conformation du domaine correspondant de la glycoprotéine d'enveloppe ou bien peut être mieux exposé après fixation sur un support solide ; les anticorps capables de réagir avec le peptide P253 seraient plus fréquemment rencontrés dans les sérums félins infectés et les 5 aminoacides situés à l'extrémité C-terminale du peptide P253 paraissent essentiels pour la reconnaissance de tous les sérums de chats infectés.

La présente invention a également pour objet un procédé de dépistage d'une infection à VIF, caractérisé en ce qu'il consiste à détecter les anticorps anti-VIF éventuellement présents dans un échantillon biologique à l'aide du peptide P253 conforme à l'invention, éventuellement fixé sur un support solide approprié, en mettant en présence ledit échantillon biologique avec ledit peptide, auxquels se lient les anticorps anti-VIF si de tels anticorps sont présents dans l'échantillon à analyser, la lecture du résultat étant révélée par un moyen approprié, notamment EIA, RIA, fluorescence.

Ce procédé permet notamment de vérifier la séroconversion des animaux vaccinés ou de procéder à des enquêtes sérologiques à visée épidémiologique.

De manière inattendue, ledit procédé permet de déterminer une infection à VIF, quelle que soit la souche (réactif universel tel que précisé ci-dessus).

Egalement de manière inattendue, lorsque ledit peptide P253 est mis en oeuvre dans un test de détection d'une infection à VIF, il apparaît plus sensible pour la détection des sérums positifs, que le peptide P237.

La présente invention a, en outre, pour objet un kit, prêt à l'emploi, pour la mise en oeuvre du procédé de dépistage d'une infection à VIF, caractérisé en ce qu'il comprend, outre des quantités utiles de tampons appropriés pour la mise en oeuvre de ladite détection, des doses appropriées du peptide P253, conforme à l'invention.

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

**EXEMPLE 1: Test ELISA comparatif entre les peptides P237 (Demande EP n°0 577 458) et P253 (conforme à l'invention).**

1) Protocole :

Une solution de 50 µl de peptides à 5 µg/ml dans du carbonate de sodium 0,1 M, pH 9,6, est adsorbée sur les puits d'une plaque de microtitration (Dynatech Immulon® 2), pendant une nuit à 4°C.

Les puits sont ensuite lavés 5 fois avec du PBS. Les sites d'adsorption résiduels des microplaques sont saturés à 37°C par incubation avec 250 µl de sérum albumine bovine 3 % dans du PBS pendant 3-4 heures.

Après 5 lavages avec du PBS contenant un tampon de lavage Tween® 0,1 % (tampon WB), 50 µl de sérum de chat dilués au 1/10ème dans un tampon PBS-BSA (1 %) Tween® 20 (0,1 %) sont incubés dans les puits, pendant 1 heure, à température ambiante.

Après lavage 3 fois avec le tampon WB, des immunoglobulines anti-chat conjuguées à la peroxydase (0,5 µg/ml) (Kirkegaard et Perry Laboratories Inc), sont ajoutées pendant une heure à température ambiante.

Après 5 lavages au PBS, le conjugué à la peroxydase lié, est visualisé avec de l'acide 2,2'-azino-bis (3-éthylbenz-thiazoline-6 sulfonique) (ABTS, Sigma) 0,5 mg/ml dans un tampon phosphate-citrate, pH 5,3, contenant du perborate de sodium 0,03 % (Aldrich).

2) <u>Résultats</u> :

Les essais sont réalisés en double.

Pour apprécier les réactions positives, une valeur limite est calculée comme suit :

$$p = 3 \times m_{NC} \ (3 \text{ fois l'absorbance moyenne des sérums négatifs contrôle } (_{NC})).$$

Les valeurs d'absorbance (ou DO pour Densité Optique) supérieures à p sont considérées comme indiquant la présence d'anticorps anti-VIF.

Les valeurs d'adsorbance inférieures à $n = 2 \times m_{NC}$ sont considérées comme indiquant l'absence d'anticorps anti-VIF.

Le Tableau I ci-après illustre les résultats obtenus avec 184 sérums de chats dont 146 sérums de chats provenant de consultation vétérinaire, 18 sérums de chats infectés expérimentalement et 20 sérums de chats sains (chat SPF).

TABLEAU I

| P237 | P253 | Nombre de Sérums (%) | |
|:---:|:---:|---:|:---|
| + | + | 110 | (59, 78) |
| - | + | 7 | (3, 80) |
| - | - | 67 | (36, 42) |
| TOTAL | | 184 | (100) |

Ce Tableau montre que le peptide P253 est significativement plus sensible pour la détection des sérums positifs que le peptide P237.

**Exemple 2: Evaluation de la sensibilité et de la spécificité du test ELISA P253.**

Pour apprécier et évaluer la sensibilité et la spécificité du peptide P253, les deux peptides P237 et P253 ont été comparés en ELISA, sur les mêmes sérums que ci-dessus, de manière à déterminer lequel des peptides permet d'obtenir la meilleure spécificité et la meilleure sensibilité, en comparaison avec un test réalisé avec un lysat de virus entier (test Petchek, IDEXX), (test virus entier) et avec un test sandwich ELISA GN, considéré comme une référence, et correspondant à un test ELISA utilisant une lectine d'origine végétale *Galanthus Nivalis* qui reconnait spécifiquement les groupes D-mannose terminaux de la glycoprotéine Env (test GN).

Les résultats sont illustrés dans le Tableau II ci-après :

TABLEAU II

| Test P237 | Test P253 | Test lysat de virus | Test *GN* ELISA | Nombre de sérums (%) |
|:---:|:---:|:---:|:---:|:---:|
| + | + | + | + | 102 (55, 43) |
| + | + | - | + | 8 (4, 35) |
| - | + | + | + | 7 (3, 80) |
| - | - | + | + | 1 (0, 54) |
| - | - | + | - | 11 (5, 98) |
| - | - | - | - | 55 (29, 9) |
| Total | | | | 184 (100) |

Il ressort de ce Tableau II que :
- dans 157 cas (85,33 %), les deux peptides donnent les mêmes résultats que le test à base de virus entier; toutefois le test P253 est significativement plus sensible que le test P237, qui présente des résultats faussement négatifs (7 sérums) ;
- aucun résultat faussement positif n'est observé, ce qui représente un intérêt majeur dans le cadre de tests vétérinaires de routine, alors que le test au virus entier révèle 11 sérums faussement positifs, ainsi que 8 sérums faussement négatifs :
  . les 11 sérums qui donnent des résultats positifs avec le test au virus entier (faux positifs) et des tests négatifs avec le test ELISA P253 ont été obtenus à partir de chats qui ne montraient aucun symptôme particulièrement évocateur d'infection à VIF, sauf quelques cas de gingivite ;
  . le test P253, interprété à la lumière des données cliniques, suggère également que les 8 sérums négatifs avec le test au virus entier correspondent à des résultats faussement négatifs, dans la mesure où parmi les 8 sérums négatifs avec le test au virus complet et positifs avec le test ELISA P253, 6 ont été obtenus à partir de chats malades, présentant de la fièvre, de la diarrhée, une gingivite et d'autres symptômes qui suggèrent une infection à VIF. Les deux autres sérums ont été obtenus à partir de chats qui montraient des désordres nerveux et comportementaux ;
- dans un seul cas, le peptide P253 présente un résultat divergent avec celui obtenu avec le test GN ; cette divergence de résultat peut s'expliquer par le fait que des mutations soient exceptionnellement intervenues dans la séquence de l'enveloppe correspondant au peptide et, par ailleurs, parfaitement constante dans tous les isolats de VIF séquencés jusqu'à aujourd'hui. Alternativement, on peut supposer que dans le sérum cité, les anticorps anti-P253 étaient complexés sous forme de complexes immuns.

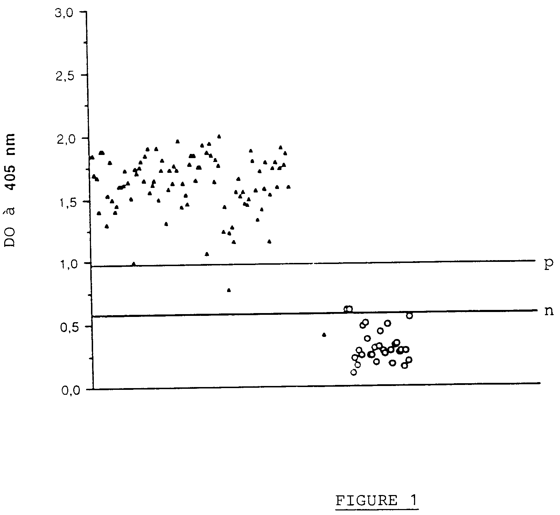

La figure 1 illustre les résultats comparatifs obtenus avec le test P253 et le test GN ; cette figure 1 montre les densités optiques obtenues avec les différents sérums testés avec le test P253 et leur répartition (chaque point représente un sérum), de part et d'autre des valeurs de densité optique p et n, telle que définies ci-dessus ; les mêmes sérums ont été testés avec le test ELISA GN : les ▲ représentent les sérums positifs au test ELISA GN tandis que les ○ représentent les sérums négatifs en ELISA GN

Ces résultats confirment :
  . que seulement un sérum positif en ELISA GN tombe en dessous de la valeur limite négative n = 2 x $m_{NC}$ du test P253 et
  . que 3 sérums se retrouvent entre les valeurs limites p et n ; la figure 1 montre que 2 de ces 3 sérums sont négatifs avec le test de confirmation ELISA GN, alors que le troisième est considéré comme positif.

Ces données correspondent donc à celles du Tableau II ci-dessus.

Le test GN précité est réalisé dans les conditions suivantes :

Ce test GN, basé sur le protocole décrit par GILLJAM (*AIDS Res. and Hum. Retrovir.*, 1993, 9, 431-438), comprend, de manière plus précise, le revêtement de plaques de microtitration (Dynatech Immulon® 2) à l'aide d'1 µg de lectine GN (Sigma, L-8275) dans 100 µl de tampon carbonate de sodium 0,1 M, pH 9,6, une nuit à température ambiante.

Après 3 lavages avec du PBS, les plaques sont incubées pendant 2 heures, à température ambiante, avec du PBS contenant du sérum de veau foetal à 10 %.

Les plaques sont alors lavées 3 fois avec le tampon WB précité.

100 µl de surnageant de cellules FL4, chroniquement infectées avec du VIF ($10^6$ cellules/ml), comprenant de l'Empigène®-BB 0,25 % (Calbiochem.) sont ensuite ajoutés à chaque puits et incubés une nuit à 4°C.

Après 3 lavages avec le tampon WB, les sérums de chats sont incubés pendant 2 heures à température ambiante (dilution 1:400 dans un tampon PBS-Tween 20 0,1 %, sérum de veau foetal 10 %).

La réaction avec une immunoglobuline anti-chat conjuguée à de la peroxydase et le développement de la coloration en présence de substrat est réalisée comme décrit ci-dessus pour le test ELISA.

Ces résultats montrent l'importance de la sélection du réactif, dans le cadre de tests vétérinaires en routine.

Le Tableau III met en valeur la sensibilité et la spécificité du test P253.

## TABLEAU III

|  | Nombre de sérums (%) |
|---|---|
| vrai positif | 117 (63,6 %) |
| vrai négatif | 66 (35,8 %) |
| faux positif | 0 |
| faux négatif | 1 (0,54 %) |
| sensibilité | 98,9 % |
| spécificité | 100 % |

Le test ELISA P253 montre une spécificité et une sensibilité significativement plus élevée que celle obtenue avec le test au lysat de virus entier ; seulement 3 sérums ont nécessité une confirmation avec le test ELISA GN.

De plus, le test P253 révèle une augmentation de la spécificité et de la sensibilité, lorsqu'on le compare à des immunoblots et à des RIPA qui conduisent souvent à des réactions croisées non-spécifiques.

**Exemple 3: Comparaison du test ELISA P253 avec un test ELISA utilisant un autre peptide synthétique.**

Ce test ELISA P253 a également été comparé avec un autre test ELISA qui utilise un peptide synthétique conjugué à la peroxydase, qui correspond au domaine immunodominant de la région transmembranaire de la protéine Env de VIF (Virachek® FIV, SYNBIOTICS CORPORATION).

Des chats contrôle négatifs SPF, des chats expérimentalement infectés et des chats naturellement infectés ont été testés avec ce peptide.

Des différences sont observées :

un sérum de chat expérimentalement infecté avec la souche LE, n'est pas réactif avec le peptide conjugué à la peroxydase, bien qu'il soit positif avec tous les autres tests. Parmi les chats naturellement infectés, 2 sérums n'ont pas été réactifs avec ledit peptide conjugué à la peroxydase bien qu'ils aient été positifs avec les tests P237, P253 et GN.

Ces résultats montrent l'importance de la sélection du réactif le plus approprié.

Contrairement aux tests P253 et GN, le peptide conjugué à la peroxydase entraîne également des résultats faussement négatif.

Le peptide P253, qui comprend 5 résidus aminoacides supplémentaires du côté C-terminal, permet d'augmenter nettement la sensibilité du test, avec une reconnaissance de pratiquement tous les sérums infectés.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

1°) Fragment peptidique issu de la protéine Env du virus de l'immunodéficience féline (VIF) de souche Wo (peptide P253), caractérisé en ce qu'il correspond aux positions 693-709 de ladite protéine Env et en ce qu'il présente la séquence suivante :
Leu-Gly-X-Asn-Gln-Asn-Gln-Phe-Phe-X-Lys-Val-Pro-Ser-Ala-, dans laquelle X représente une cystéine ou une sérine, conformément aux séquences ID N° 1 à 4.

2°) Procédé de dépistage d'une infection à VIF, caractérisé en ce qu'il consiste à détecter les anticorps anti-VIF éventuellement présents dans un échantillon biologique à l'aide du peptide selon la revendication 1, éventuellement fixé sur un support solide approprié, en mettant en présence ledit échantillon biologique avec ledit peptide, auxquels se lient les anticorps anti-VIF si de tels anticorps sont présents dans l'échantillon à analyser, la lecture du résultat étant révélée par un moyen approprié, notamment EIA, RIA, fluorescence.

3°) Kit ou trousse de diagnostic, prêt à l'emploi, pour la mise en oeuvre du procédé de dépistage d'une infection à VIF, caractérisé en ce qu'il comprend, outre des quantités utiles de tampons appropriés pour la mise en oeuvre de ladite détection, des doses appropriées d'un peptide selon la revendication 1.

FIGURE 1

# EP 0 688 790 A1

**Office européen des brevets**

## RAPPORT DE RECHERCHE EUROPEENNE

Numero de la demande
**EP 95 40 1316**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,Y | EP-A-0 577 458 (CENTRE NATIONAL DE LA RECHERCHE SCIENTIFIQUE (CNRS)) <br> * figure 1A * <br> * exemples 4,5 * <br> * revendications * <br> --- | 1-3 | C07K14/155 <br> G01N33/569 <br> G01N33/68 |
| Y | WO-A-93 01304 (SYNBIOTICS CORPORATION) <br> * le document en entier * <br> --- | 1-3 | |
| A | RESEARCH IN VIROLOGY, <br> vol. 144, no. 3, Mai 1993 PARIS, FRANCE, <br> pages 209-218, <br> A. AVRAMEAS ET AL. 'Serological diagnosis of feline immunodeficiency virus infection based on synthetic peptides from Env glycoproteins.' <br> * le document en entier * <br> --- | 1-3 | |
| A | JOURNAL OF VIROLOGY, <br> vol. 67, no. 2, Février 1993 BALTIMORE MD, TATS UNIS, <br> pages 664-672, <br> G. PANCINO ET AL. 'B epitopes and selection pressures in feline immunodeficiency virus envelope glycoproteins.' <br> * le document en entier * <br> --- | 1-3 | **DOMAINES TECHNIQUES RECHERCHES (Int.Cl.6)** <br> C07K <br> G01N |
| A | WO-A-92 22573 (ST. VINCENT'S INSTITUTE OF MEDICAL RESEARCH, LTD.) <br> * le document en entier * <br> --- <br> -/-- | 1-3 | |

Le présent rapport a été établi pour toutes les revendications

| Lieu de la recherche | Date d'achèvement de la recherche | Examinateur |
|---|---|---|
| LA HAYE | 11 Octobre 1995 | Nooij, F |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................................
& : membre de la même famille, document correspondant

EPO FORM 1503 03.82 (P04C02)

**Office européen
des brevets**

**RAPPORT DE RECHERCHE EUROPEENNE**

Numero de la demande

EP 95 40 1316

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| P,X | VETERINARY MICROBIOLOGY, vol. 45, no. 2-3, 1995 AMSTERDAM, NL, pages 259-267, P. SIBILLE ET AL. 'Comparison of serological tests for the diagnosis of feline immunodeficiency virus infection of cats.' * le document en entier * | 1-3 | |
| | | | **DOMAINES TECHNIQUES RECHERCHES (Int.Cl.6)** |

Le présent rapport a été établi pour toutes les revendications

| Lieu de la recherche | Date d'achèvement de la recherche | Examinateur |
|---|---|---|
| LA HAYE | 11 Octobre 1995 | Nooij, F |

## LISTE DE SEQUENCES

(1) INFORMATIONS GENERALES:

(i) DEPOSANT:
(A) NOM: CENTRE NATIONAL DE LA RECHERCHE SCIENTIFIQUE-C.N.R.S.
(B) RUE: 3 RUE MICHEL-ANGE
(C) VILLE: PARIS
(E) PAYS: FRANCE
(F) CODE POSTAL: 75794 CEDEX 16

(ii) TITRE DE L' INVENTION: FRAGMENT PEPTIDIQUE SPECIFIQUE DU VIRUS DE L'IMMUNODEFICIENCE FELINE (VIF) ET SON UTILISATION COMME REACTIF DE DIAGNOSTIC.

(iii) NOMBRE DE SEQUENCES: 4

(iv) FORME DECHIFFRABLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(2) INFORMATIONS POUR LA SEQ ID NO: 1:

(i) CARACTERISTIQUES DE LA SEQUENCE:
(A) LONGUEUR: 15 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 1:

Leu Gly Cys Asn Gln Asn Gln Phe Phe Cys Lys Val Pro Ser Ala
1               5                 10                 15

(2) INFORMATIONS POUR LA SEQ ID NO: 2:

(i) CARACTERISTIQUES DE LA SEQUENCE:
(A) LONGUEUR: 15 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 2:

Leu Gly Ser Asn Gln Asn Gln Phe Phe Ser Lys Val Pro Ser Ala
1           5           10          15

(2) INFORMATIONS POUR LA SEQ ID NO: 3:

(i) CARACTERISTIQUES DE LA SEQUENCE:
   (A) LONGUEUR: 15 acides aminés
   (B) TYPE: acide aminé
   (C) NOMBRE DE BRINS: simple
   (D) CONFIGURATION: linéaire

(ii) TYPE DE MOLECULE: peptide

(xi) DESCRIPTION DE LA SEQUENCE: SEQ ID NO: 3:

Leu Gly Cys Asn Gln Asn Gln Phe Phe Ser Lys Val Pro Ser Ala
1           5           10          15

(2) INFORMATIONS POUR LA SEQ ID NO: 4:

(i) CARACTERISTIQUES DE LA SEQUENCE:
   (A) LONGUEUR: 15 acides aminés
   (B) TYPE: acide aminé
   (C) NOMBRE DE BRINS: simple
   (D) CONFIGURATION: linéaire

(ii) TYPE DE MOLECULE: peptide

(xi) DESCRIPTION DE LA SEQUENCE: SEQ ID NO: 4:

Leu Gly Ser Asn Gln Asn Gln Phe Phe Cys Lys Val Pro Ser Ala
1           5           10          15